# EUROPEAN PATENT APPLICATION

(11) **EP 1 550 466 A1**
(43) Date of publication of application: **06.07.2005**
(21) Application number: 03792811.6
(22) Date of filing: 22.08.2003
(51) Int. Cl.: A61L 2/26

(54) **STERILE BAG**

(30) Priority: 22.08.2002 JP 2002241780; 11.09.2002 JP 2002265274
(71) Applicant: Kao Corporation, Tokyo 103-8210 (JP); FUJIMORI KOGYO CO., LTD., Tokyo 103-0002 (JP)
(72) Inventor: MATSUMOTO, Kiyoshi KAO CORPORATION, Tokyo 131-8501 (JP); ABE, Keiji KAO CORPORATION, Tokyo 131-8501 (JP); SUTOH, Teiko FUJIMORI KOGYO CO., LTD., Tokyo 103-0002 (JP); INAGAKI, Tsuyoshi FUJIMORI KOGYO CO., LTD., Tokyo 103-0002 (JP); KANAYA, Kikuji FUJIMORI KOGYO CO., LTD., Tokyo 103-0002 (JP)
(74) Representative: Fuhlendorf, Jörn, Dipl.-Ing.
(86) International application number: PCT/JP2003/010658
(87) International publication number: WO 2004/018004

(57) **Abstract**

A sterilization bag that is favorable for storing medical implements having sharp tips such as scalpels and forceps and can effectively suppress the occurrence of unwanted opening or damage due to contact with sharp tips is provided.

The sterilization bag is formed by adhering the side edges and lower ends of a gas-permeable material and a synthetic resin film. The sterilization bag has, in its lower end portion or vicinity thereof, a damage-prevention means that prevents damage of at least one selected from the gas-permeable material, the synthetic resin film, and the adhered portion of a gas-permeable material and synthetic resin film by catching or receiving the tips of stored articles. Specific embodiments include a bag having a bulging portion obtained by mountain folding and valley folding a side surface film of synthetic resin in the lower end portion of the sterilization bag or vicinity thereof, and a bag with a laminated protective component made of synthetic resin film.

## Description

### Technical Field

The present invention relates to a sterilization bag that excels in handling, more particularly, to a sterilization bag that can be suitably used for storing and sterilizing medical instruments having sharp edges.

### Background of the Art

It is necessary to sterilize instruments used in surgery and medical treatment prior to use. When stored contents are material solid objects such as mechanical parts and various instruments, peel-off type bags (in which the bag is peeled and opened) and films that are easily torn and opened are employed. For example, in sterilization methods practiced in hospitals and the like, objects to be sterilized, such as large tweezers, scalpels, or forceps, are placed within a sterilization bag at least having a region that is gas-permeable but impermeable to germs and bacteria. After tightly sealing the bag, sterilization is performed with a method using high-temperature steam, plasma, ethylene oxide gas (EOG) and the like. From after the sterilization of the instruments until use thereof in surgery and the like, the instruments are stored within the bag, and upon surgery or treatment, the bag is opened and the instruments are used. For this reason, in order to enable doctors and medical practitioners in hospitals using gloves to open the bags easily, the bags for sterilization are made of two rectangular thinly-formed substances such as sheets or films (hereafter these are referred to as "film") in front and reverse side thereof . These films are adhered so as to be peelable, or made so as to tear easily. When opening the bags, a tearing method or a so-called peel-open form where peeling the two films is performed to open the films is generally employed.

Conventionally, after putting medical instruments having sharp tips such as scalpels and forceps in such a sterilization bag and then sterilizing it, it is often the case that the bag having the instruments therein is disposed in a container, such as a forceps stand, with the tips downwards, in order to obtain easy use of the instruments. As mentioned above, sterilization bags generally have two films that are heat sealed so as to be peelable. For this reason, when these types of medical instruments are stored in the sterilization bag, or when, after sterilization, the bag is disposed in the forceps stand, the tip portions of the medical instruments contact the heat-sealed portion of the lower end of the sterilization bag. There have been problems where the heat-sealed portion thus peels and opens. Further, when heat sterilizing such as autoclave sterilization (AC sterilization) is performed for a sterilization bag in which one of base materials serving as a gas-permeable substrate is paper and in which the other is a synthetic resin film, there is a tendency for only the synthetic resin film to shrink and curl, making it easier for the tip portions of the medical instruments to contact the paper substrate in the lower end of the sterilization bag. There has thus been a tendency for the paper substrate to be damaged.

Moreover, it is necessary to use a film of paper or non-woven fabric that is gas-permeable but impermeable to germs and bacteria. However, when medical instruments having sharp tips or uneven portions such as large tweezers and forceps are put by sliding into the bag, the tips catch in spaces between the fibers of the paper or non-woven fabric, or the uneven portions pierce the bag, whereby pinholes may form and the sterilization bag may become unusable.

In order to prevent this unwanted damage to sterilization bags, several proposals have been made. For example, a sterilization bag for these kinds of objects having sharp tips to be sterilized has a bottom portion that is formed with bottom film made of synthetic resin and having a boat-like shape (e.g., refer to Patent Publication 1 shown below). In this type of sterilization bag, the synthetic resin film of the bottom surface is mountain folded (convexly folded) when not in use, and when objects to be sterilized are put by sliding into the bag, the bottom of the bag opens to take the form of a so-called standing pouch. When multiple forceps and the like are stood up and kept therein, there is the advantage in that the bag saves space. Nonetheless, when forceps and the like are inserted, it is easy for the tips of the inserted forceps to come into contact with the portion of junction with the non-woven fabric, and the problem of accidental damage easily occurring still remains.

### (Patent Publication 1) Japanese Patent Application Laid-Open (JP-A) No. 8-168518

### Disclosure of Invention

### Problems to be Solved by the Invention

The present invention was created in order to solve these kinds of problematic points. The purpose of the present invention is to provide a sterilization bag for storing medical instruments having sharp tips such as scalpels and forceps wherein, even when heat is applied to the sterilization bag to perform sterilization, the sterilization bag can effectively suppress unwanted opening of the bag due to contact with the sharp tip portions, or the occurrence of damage accidents.

### Means of Solving the Problems

The sterilization bag of the present invention is a sterilization bag in which an opening portion is left in the upper portion and the side edges and lower ends of a gas-permeable material and synthetic resin film are adhered. The sterilization bag has, in the lower end portion of the sterilization bag or vicinity thereof, a damage prevention means for preventing damage of at least one selected from the gas-permeable material, the synthetic resin film, and the adhered portion of the gas-permeable material and the synthetic resin film, and the damage prevention means prevents the damage by catching or receiving the tips of the stored articles.

This type of damage prevention means is preferably one having, in the lower end portion of the sterilization bag or vicinity thereof, a shape that eases the stress applied by articles, which are inputted in the bag as objects to be sterilized, and that receives the tips of the stored articles. Further, the damage prevention means is also preferably a means with a protective component made of a high-strength material and fixed and disposed on the interior side of the lower end portion of the sterilization bag or vicinity thereof, which means receives the tips of the stored articles and prevents damage.

Hereafter, detailed explanations will be given with regard to the preferable embodiments of the sterilization bag of the present invention. The first preferable embodiment of the sterilization bag of the present invention has a bulging portion in the lower end portion of the bag or vicinity thereof, which bulging portion prevents damage by receiving the tips of the stored articles.

Namely, the sterilization bag of the first embodiment of the present invention is made by adhering the sides and lower ends of a gas-permeable material and a synthetic resin film to each other so that an opening portion is left in the upper portion. The damage prevention means is a bulging portion formed by mountain folding and valley folding (concavely folding) the synthetic resin film and disposed in the lower end portion of the sterilization bag or vicinity thereof, and the bulging portion prevents damage by receiving the tips of the stored articles.

Moreover, the damage prevention means in the sterilization bag of the second embodiment of the present invention utilizing shape is a bulging portion formed by mountain folding the synthetic resin film and disposed in the lower portion of the sterilization bag, and is characterized in that the mountain foldedfolding line of the side surface film is positioned in the lowermost end of the sterilization bag.

Preferable embodiments of such a bulging portion are as follows. The side edges of the bulging portion are obliquely adhered so that the lower end is shorter than the opening in the upper end portion. The slope of the obliquely adhered portion has an intersecting angle of an adhered portions in the side edge of the bulging portion and the folding line formed from a mountain foldof the side surface film made of synthetic resin, and this angle is within the range of 120° to 150°.

The outer sides of the adhered portions of the bulging portion may be cut and removed.

As discussed previously, the adhered portions of the sterilization bag are heat sealed or lightly adhered to the extent that they are easily peelable to enable for the contents to be emptied from the interior at use. For this reason, when, for example, a sharp tip directly contacts a heat-sealed portion, the tip progresses to stab the sealed region and the sealed portion peels. By providing a bulging portion of synthetic resin film in the lower portion of the sterilization bag in the first and second preferable embodiments of the present invention, in cases where medical instruments having sharp tips are put by sliding into the bag, the tips are retained in the mountain folded portion of the bulging portion, and do not contact the adhered portions. Therefore, undesired opening of the adhered portions can be effectively suppressed. Further, when one of base materials is paper and the other is a synthetic resin film, and when heat sterilization is performed with, for example, autoclave sterilization (AC sterilization), situations often arise where only the synthetic resin film shrinks and curls and it becomes easier for the tips of the medical instruments to come into contact with the paper material in the lower end of the sterilization bag. Even in these cases, the problem of the paper material being damaged does not occur due to the present embodiments.

Here, by obliquely sealing the bulging portion so that the lower end of the side edges of the bulging portion becomes narrow, the bulging portion becomes easy to open and the lower end easily forms a gusset. For this reason, the tips of the medical instruments reaching the adhered portion of the corner section or the concentration of the weight of the medical instruments and external force through the tips of the medical instruments on the corner section of the bag can be more effectively prevented.

In the sterilization bag of the present invention, since one of the base materials is made of a gas-permeable material such as paper or a non-woven fabric (hereafter referred to as "sterilization paper" when appropriate) and the other base material is made of a synthetic resin formed into a film (synthetic resin film), the sterilization efficiency and adhesion of the both materials of the bag are excellent. It is preferable from the point of visibility of the objects to be sterilized that the synthetic resin film is transparent.

Next, another preferable embodiment of the present invention will be explained, in which damage prevention means is formed by fixing a protective component made of a high-strength material.

The sterilization bag of the third embodiment of the present invention is a sterilization bag made by adhesion of the side edges and the lower ends of a gas-permeable material and a synthetic resin film so that an opening portion is left in the upper portion. The damage prevention means is a protective component arranged on the inner surface of the lower end portion of the sterilization bag or the vicinity thereof, and it prevents damage by receiving the tips of the stored articles.

This protective component is arranged on the interior side of the sterilization bag, and can protect not only the gas-permeable material, but also the synthetic resin film and the adhered portions of the gas-permeable material and the synthetic resin film.

The protective component can be a molded article made of synthetic resin or a component formed into a film. Further, when it protects only the gas-permeable material, it can be multilayered and arranged in the lower end portion and vicinity thereof. Furthermore, when it also protects the synthetic resin film (the other side surface film), it can be a protective component made of multiple film materials, a protective component formed into a tube shape, or a component having a closed bottom or a bag-shaped component formed by mountain folding a film protective component into an L or U-shape.

When this kind of protective component is bag-shaped, it can be formed by extending and mountain folding the synthetic resin film, or mountain folding a third synthetic resin film different from the side surface film.

This type of protective component can be heat sealed and fixed to at least one of the gas-permeable material and the synthetic resin film, and it is preferable that it be fixed to at least the gas-permeable material. It is more preferable that it be fixed by heat sealing to both the gas-permeable material and the synthetic resin film. Furthermore, when it is also fixed in the lower heat-sealed portion, the adhesion strength of the lower sealed portion increases, and peeling of the lower sealed portion by sharp tips can be more effectively prevented.

For the protective component, high-strength materials such as synthetic resin films may be generally used. However, it is preferable to use non-woven fabric having gas permeability or synthetic resin films having many perforated holes, because these can be favorably used with autoclave sterilization and the like, which requires moisture permeability.

All of these sterilization bags can be favorably used for sterilizing one type or two or more types of medical instruments selected from the group consisting of scalpels, tweezers, forceps and scissors.

Conventionally, in the gas-permeable paper or non-woven fabric used as a material that is permeable to gases but impermeable to bacteria and germs in the sterilization bag, its micro-spaces between the fibers ensure gas permeability. This type of material is problematic in that, when the tips and uneven portions of objects to be sterilized, such as large tweezers, directly contact the paper or non-woven fabric in a region where the base material is exposed on the interior side at the time that the objects are inserted, the tips or uneven portions proceed piercing the micro-spaces of the fiber region. Such objects form pinholes or catch on the tips of the fibers and the bonding point of the fibers, thus forming tears. This type of tendency is particularly marked with items such as large tweezers, which add stress between the fibers due to their own weight. In the present invention, by arranging a protective component that protects at least the gas-permeable material on the interior side of the sterilization bag, the surface of the gas-permeable material having micro-spaces formed by the exposure of fibers on the surface is covered and protected by the protective component, which is made from synthetic resin material or synthetic resin film and the like and has high strength against piercing and high heat sealing strength. Even in cases where medical instruments having sharp tips are put sliding into the bag, the tips and uneven portions do not pierce due to the protective component, and the occurrence of undesirable pinholes and tearing the gas-permeable material can be effectively suppressed.

This type of protective component, in a preferred embodiment, has a closed bottom or a bag-shaped form, with an opening left in the upper portion. That is, the cross-section thereof is an L shape or U shape. In this case, it can protect not only the gas-permeable material, but also areas such as the surface of the synthetic resin film that is the other side surface film and their adhered portions. It not only protects the side surfaces from irregularities, but also effectively eases peeling of the adhered portions due to stress in the perpendicular direction upon inputting. Therefore, it more effectively prevents damage from the tips and uneven portions of medical instruments and undesired opening.

### Effects of the Invention

Due to the sterilization bag of the present invention, even when medical instruments having sharp tips or uneven portions, such as large tweezers, scalpels and forceps, are stored therein, an effect is achieved where the occurrence of undesired accidental opening and damage due to contact with the sharp tips can be effectively suppressed.

### Brief Description of the Drawing

Fig. 1A is a front view showing a first embodiment of the sterilization bag of the present invention, and Fig. 1B is a cross-sectional diagram of the same.
Fig. 2 is a diagram schematically showing the process of manufacturing the sterilization bag of the first embodiment of the present invention.
Fig. 3A is a front view showing a second embodiment of the sterilization bag of the present invention, and Fig. 3B is a cross-sectional diagram of the same.
Fig. 4 is a diagram schematically showing the process of manufacturing the sterilization bag of the second embodiment of the present invention.
Fig. 5 shows a perspective view including a cutaway portion of the sterilization bag of a third embodiment of the present invention where a protective film is laminated on the surface of sterilization paper.
Fig. 6A shows a cross-sectional view of the films that compose the lower end portion of the sterilization bag of the third embodiment of the present invention where a protective film is laminated on the surface of sterilization paper, and Fig. 6B shows a cross-sectional view of the sterilization bag formed by heat adhering these films.
Fig. 7A shows a cross-sectional view of the films that compose the lower end portion of the sterilization bag where the protective film of sterilization paper surface is an extended side surface film, and Fig. 7B shows a cross-sectional view of the sterilization bag formed by heat adhering these films.
Fig. 8 shows a perspective view including a cutaway portion of the sterilization bag where a protective film is laminated on sterilization paper surface and a side surface film to form a V-shaped bag-shaped portion between the sterilization paper and the side surface film.
Fig. 9A shows a cross-sectional view of the films forming the lower end portion of the sterilization bag where a protective film is laminated on sterilization paper surface and a side surface film to form a V-shaped bag-shaped portion between the sterilization paper and the side surface film, and Fig. 9B shows a cross-sectional view of the sterilization bag formed by heat adhering these films.
Fig. 10A shows a cross-sectional view of the films forming the lower end portion of the sterilization bag where a protective film is laminated on sterilization paper surface and a side surface film to form a V-shaped bag-shaped portion between the sterilization paper and the side surface film, and Fig. 10B shows a cross-sectional view of the sterilization bag formed by heat adhering these films.
Fig. 11 shows a cross-sectional view of a sterilization bag where a protective component obtained by heat sealing a non-woven cloth to a synthetic resin film so as to form a V-shaped bag-shaped portion between sterilization paper surface and a side surface film.

### Best Mode for Carrying Out the Invention

Below, the present invention will be explained in detail.

First, examples will be raised and explained with regard to the preferable embodiments of the sterilization bag of the present invention. The sterilization bag of the present invention is formed by peelably adhering two base materials (side surface films) and the adhering the two base materials is easily achieved by using, as one of the two base materials, a gas-permeable material such as paper (sterilization paper) and, as the other, a synthetic resin film having a heat-sealing property. In order to control the adhesive strength and the peelability with more certainty, a publicly known method can be adopted. For example, easily peelable adhesive can be used. Alternatively, a thermoplastic resin non-woven fabric serving as one of the base materials and a thermoplastic transparent synthetic resin film that exhibits a proper degree of adhesive strength and that serves as the other of the materials can be used together, and/or a seal layer can be laminated on the sterilization paper without harming the gas permeability of the sterilization paper, and the both materials can be heat-sealed and adhered to each other

Since sterilization is conventionally performed with high-temperature moisture, ethylene oxide gas (EOG), plasma and the like, it is necessary that the sterilization paper used in the sterilization bag of the present invention be a material that allows their effective elements (gases) to pass through while not allowing germs to pass through. The material for this type of sterilization paper can be paper but it is preferable to select and use a non-water-absorbing material such as polyester, polyvinyl chloride, polyethylene, polypropylene or fluorinated material. A specific example thereof is gas-permeable polyethylene or polypropylene non-woven fabric.

Further, for the synthetic film that is the other material, a film shaped by fusion extruding, flow extending or calendaring a synthetic resin is used from the standpoint of cost, strength, and the visibility of the contents. The synthetic resin film is not particularly limited. However, preferable synthetic resins are those having a heat-sealing property because they are easy to handle at the time of bonding. Among these, from the standpoint of heat-sealing property, polyolefins such as polyethylene and polypropylene, and polyvinyl chlorides are preferable. The synthetic resin film does not necessarily have to be of a single-layer structure; depending on the objective, a multi-layer structure can be used. For example, when composing the side surface film with a synthetic resin film having a heat-sealing property, it is enough that one surface of the side surface film has a heat-sealing property. Hence, it is preferable to laminate a biaxially oriented film such as polyethyleneterephthalate (PET), nylon, PP and the like on the other surface because the strength against piercing and impact increases. Two or more biaxially oriented films can be laminated

The thickness of the synthetic resin side surface film can be properly selected in accordance with the purpose, however, it generally ranges from 30 to 100µm.

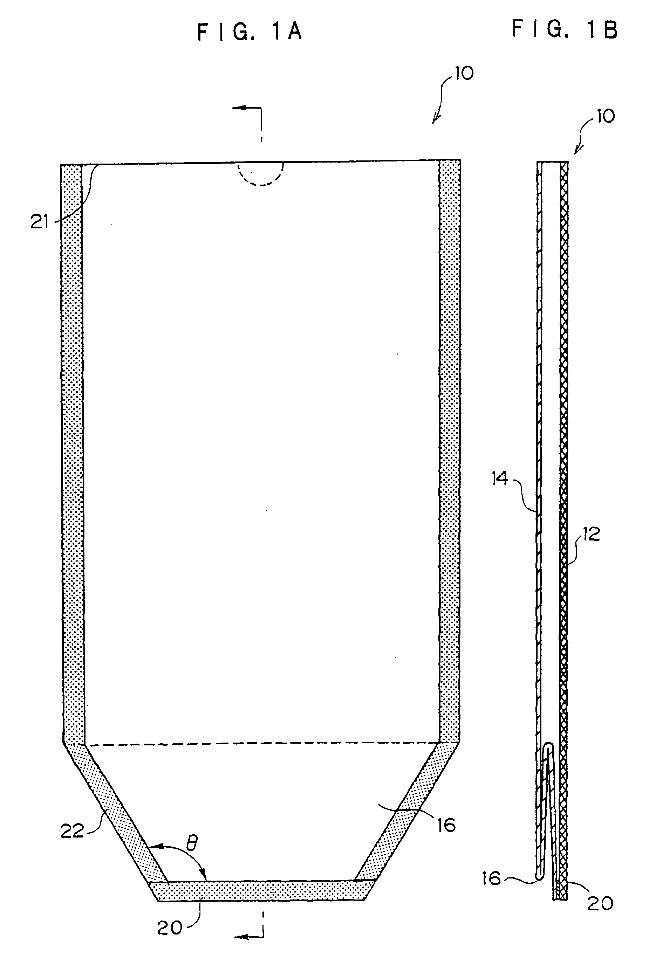

Fig. 1A is a front view showing one favorable embodiment of a sterilization bag of the first embodiment of the present invention, and Fig. 1B is a cross-sectional diagram of the same.

One of the materials (side surface material) 12 of the sterilization bag 10 is sterilization paper. The other material, i.e., the side surface film 14 made of synthetic resin, has a bulging portion 16 formed by mountain folding and valley folding this film 14 so that the film 14 is faced towards the exterior side. The upper end 18 of the sterilization bag 10 has an opening to enable insertion of objects to be sterilized and the lowermost end portion is tightly sealed due to heat sealing of the sterilization paper 12 and the synthetic resin film 14. The heat-sealed portion 20 of the lowermost end is formed in the region that protrudes further below the bulging portion 16. As shown in Fig. 1A, the side edge portions of the bulging portion are sealed at a slant so that the lower end side becomes narrow. It is preferable that the folding line of the film in the bulging portion formed by mountain folding the synthetic resin film be positioned at the same line as the lowermost end portion of the sterilization bag or lower than it. However, when forming the bag having the heat-sealed portion in the lowermost end portion of the bag, the lowermost end portion of the bulging portion becomes a hindrance in the formation process of the sealed portion. If considering facility of manufacturing with this shape, it is preferable that the lowermost end portion of the bulging portion be positioned higher by the width of the sealed portion than that of the heat-sealed portion.

It is preferable that the oblique adhered portions 22 of the side edge portions of the bulging portion be obliquely adhered so that the lower end side is shorter than the opening side 21. More specifically, they are preferably adhered at a slant such that the intersecting angle (indicated as θ in Fig. 1A) of one side edge portion of the bulging portion and the folding line formed by mountain folding the synthetic resin film is within the range of 120° to 150°. In such a case, even if the tips of the stored medical instruments come to settle in the corner portion of the wrapping bag, it becomes difficult for them to rest in the corner portion. Hence, strikes on the side edge adhered portions of the wrapping bag in the corner portions by the tips of the medical instruments can be avoided. Further, by configuring in this manner, the bulging portion is easily opened and the lower end can easily form a gusset. Therefore, the reaching of the tips of medical instruments in the corner portions of the adhered portions, or the concentration of the weight of the medical instruments and external force in the corner portions of the wrapping bag through the tips of the medical instruments can be more effectively prevented. In the present embodiment shown in Fig. 1A, the exterior side of the oblique bulging portion side end heat-sealed portion 22 has been cut and removed. However, as long as it has this kind of oblique adhered region 22, it is not always necessary for the side end portion to be cut and removed.

In the sterilization bag 10 of the present embodiment, when the upper end of the opening portion is opened to input the objects to be sterilized therein, the bulging portion 16 forms a gusset and the tips of the inserted objects to be sterilized are guided to the bulging portion 16 made from the synthetic resin film, due to such construction and the surface smoothness of the synthetic resin film. The tips contact the mountain folded end portion of the bulging portion 16 and are retained therein.

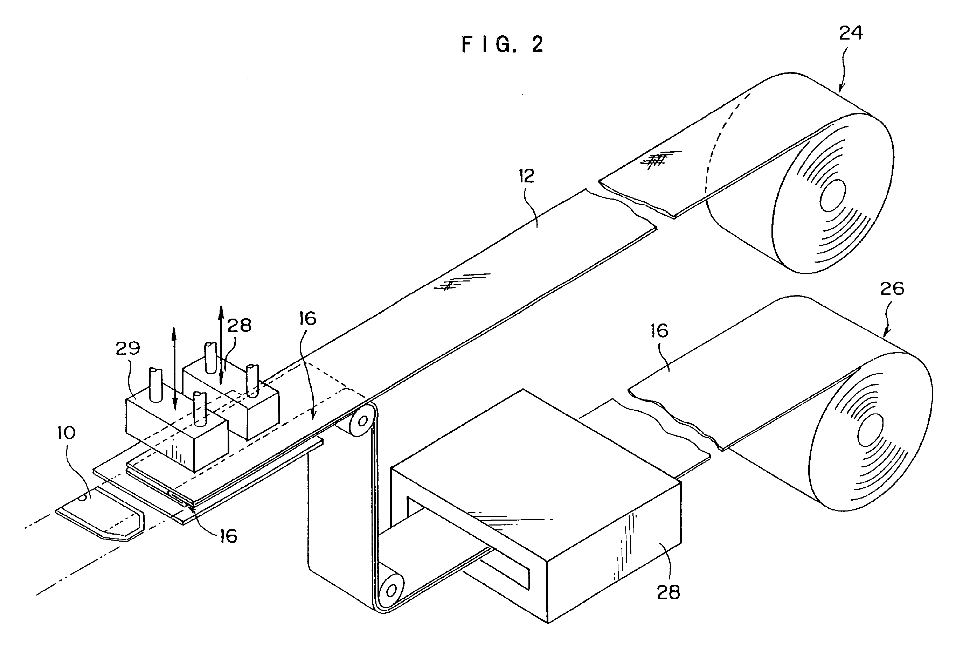

The sterilization bag of this embodiment can be easily manufactured by a conventional method. Fig. 2 is a diagram schematically showing the process for manufacturing the sterilization bag of this first embodiment. Sterilization paper 12, which is one of base materials, is supplied from a roll 24 and a synthetic resin film 14 is supplied from a roll 26. When the film 14 supplied from the roll 26 passes through a folding board unit 28, one side end of the film 14 is mountain folded and valley folded and an N-shaped bulging portion 16 is formed inward from the furthest side end portion of the synthetic resin side surface film. That is, as shown previously in Fig. 1B, the tip mountain folded portion of this bulging portion 16 is formed so as to be further towards the interior side than the furthest side end portion of the synthetic resin film. The sterilization paper 12 and the synthetic resin film 14 in which the bulging portion 16 is formed are layered and the layered portion is on the side where the bulging portion is formed and positioned outward than the bulging portion 16. In the layered portion, a part of the synthetic resin film which part protrudes from the bulging portion and does not overlap with any other part of the synthetic resin film is heat-sealed to one end portion of the sterilization paper 12 with a heating means such as a heat-seal bar 28. The other end portions of the sterilization paper and the synthetic resin film become the opening portion to enable the insertion of the objects to be sterilized.

In this manner, after providing the heat-sealed portion 20 serving as the lowermost end of the sterilization bag 10, the oblique sealed portions of the bulging portion side ends and the side end heat-sealed portions of the sterilization bag to be formed are formed through a conventional method, and portions corresponding to the side ends of the sterilization bag to be formed are cut by a cutter, and the sterilization bag 10 is obtained. The heat sealing and cutting of the side ends, as shown in Fig. 2, is performed with a cutter 29 provided with a sealing means and a cutting means, and sealing and cutting can be performed simultaneously. Alternatively, sealing and cutting can be performed sequentially with separate components.

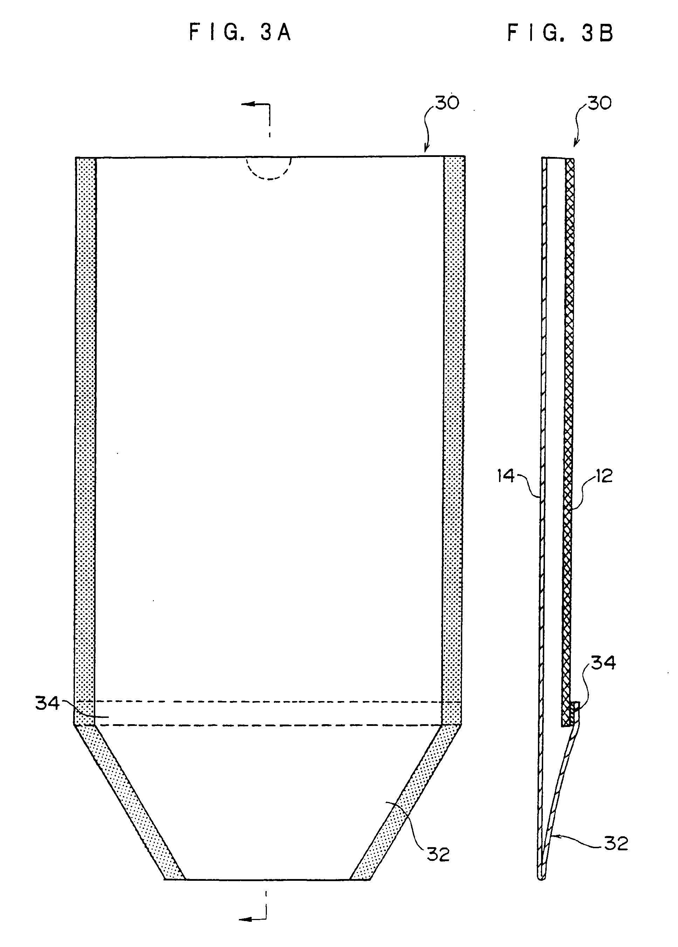

Fig. 3A is a front view of another preferable embodiment of the sterilization bag that is a second embodiment of the present invention. Fig. 3B is a cross-sectional diagram thereof.

Sterilization paper 12, which is the gas-permeable material used in a sterilization bag 30, and synthetic resin film 14 can be the same as those used in the first embodiment. In the present embodiment, the folding line of an bulging portion 32 which is mountain folded so that it is faced towards the exterior side is positioned at the lowermost end portion of the sterilization bag 30. An adhesion portion 34 of the sterilization paper 12 and the synthetic resin film 14 is positioned at the side surface portion in the vicinity of the lower end of the sterilization bag 30. In this embodiment, the lowermost portion of the sterilization bag 30 is formed from the synthetic film 14, and the heat-sealed portion 34 is positioned on the side surface of the bag 30. Therefore, even if objects to be sterilized having sharp tips are inserted into the bag 30, these can be smoothly stored without the tips catching on the heat-sealed portion 34 and are retained by the bulging portion 32 of the synthetic resin film, and there is no fear of the tips contacting the adhered region or vicinity thereof in a direction that would cause peeling the sealed portion 34 .

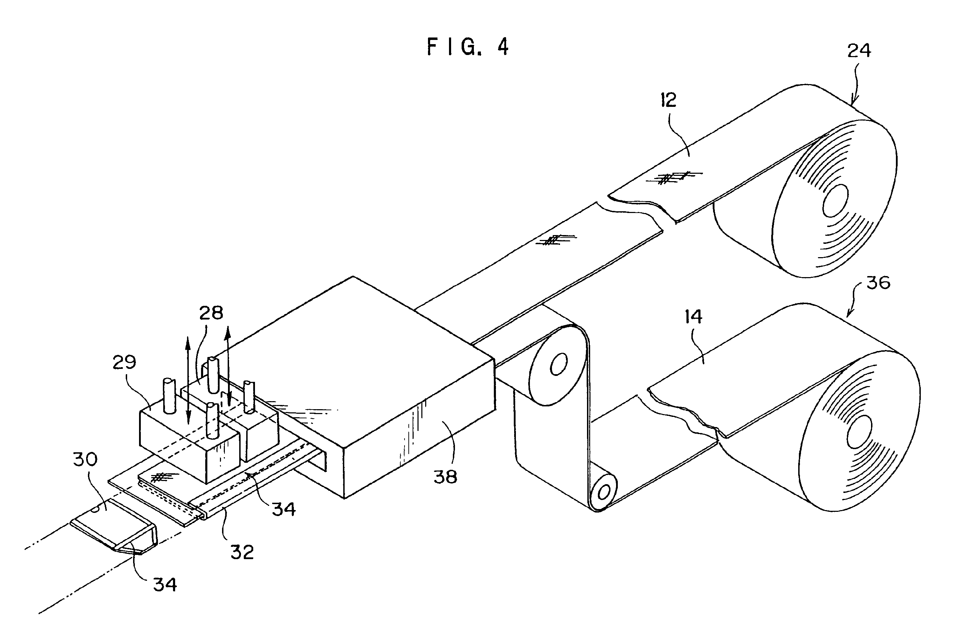

Fig. 4 is a diagram schematically showing the process of manufacturing the sterilization bag of the second embodiment. The sterilization paper 12, which is one of the base materials, is supplied from a roll 24 and the other material, the synthetic resin film 14, is supplied from a roll 36. When the synthetic resin film 14 is passing through a folding board unit 38, the side end of the film 14 supplied from the roll 36 is mountain folded and a U-shaped bulging portion 32 is formed at the furthest side end of the synthetic resin film. That is, as shown previously in Fig. 3B, the tip of the mountain folded portion of this bulging portion 32 is formed so as to be positioned at the lowermost end portion of the sterilization bag 30 and the heat-sealed portion 34 is formed at the interior side thereof. The synthetic resin film 14 folded to form the bulging portion 32 and the sterilization paper 12 are layered. However, in order to prevent deforming of parts of the synthetic resin film 14 other than the end of the U-shaped bulging portion and the occurrence of unwanted sealing by the heat when forming the heat-sealed portion 34, the end of the synthetic resin film 14 which end is to adhere to the sterilization paper 12 is layered over the sterilization paper 12 so that a heat-blocking plate is sandwiched between the U-shaped bulging portion 34. In this region, the end of the synthetic resin film 14 mountain folded into a U-shape and the sterilization paper 12 are heat sealed with a heating means such as a heat-seal bar 28. The other end portions of the synthetic resin film and the sterilization paper become the opening to enable the insertion of objects to be sterilized.

In this manner, the lowermost end of the sterilization bag 30 is the mountain folded portion of the synthetic resin film 14 and the bulging portion 32. After the folded back tip portion of the synthetic resin film 14 and the sterilization paper 12 are adhered, the oblique sealed portions of the bulging portion side ends and the side end heat-sealed portions of the sterilization bag to be formed are formed through a conventional method, and portions corresponding to the side ends of the sterilization bag to be formed are cut by a cutter, and the sterilization bag 30 is obtained. The heat sealing and cutting of the side ends, as shown in Fig. 4, is performed with a cutter 29 provided with a sealing means and a cutting means, and sealing and cutting can be performed simultaneously. Alternatively, sealing and cutting can be performed sequentially with separate components, as in the first embodiment.

In the second embodiment, the tips of the inputted objects to be sterilized are retained by the synthetic resin film of the lowermost end of the sterilization bag, so it is not necessary to specially provide a gusset in the bulging portion 32. Nonetheless, in cases where the objective is to accommodate large contents such as inserting large objects to be sterilized, it is possible to provide gussets at the side end portions of the sterilization bag.

The shape of the sterilization bag of the present invention is not limited to these embodiments. For example, it is not necessary for the whole synthetic resin film to be configured of one sheet, it can be configured so that multiple films are bonded to each other. By configuring in this manner, an article can be used in which two layers or more of high-strength resin film such as PET or nylon which are laminated in the bottom surface of vicinity thereof which tends to contact the sharp tips of objects to be sterilize, or in which a gas-permeable non-woven fabric excelling in strength is used as the base film in the bottom surface or the vicinity thereof, and in which a single layer of high-strength resin film can be used in other portions. This configuration can be advantageously applied from a cost perspective. Further, by configuring a sterilization bag in this manner, the sterilization bag is advantageous in that publicly known and widely-used bag producing machines can be used to make an article, by mountain folding the bottom surface to form a bulging portion, in contrast to the valley folding in producing a standing pouch, by inserting a board unit into the bulging portion and by heat sealing the bulging portion and the sterilization paper.

In the first or second embodiment of the present invention, when objects to be sterilized are inserted into the sterilization bag, the tips of the objects are retained in the bulging portion of the synthetic resin side surface film and they do not proceed to the adhered portion of the two side surface films. Accordingly, undesired damage or opening of the bag when inserting and retaining objects to be sterilized having sharp tips such as scalpels, forceps and scissors can be effectively prevented. For this reason, the range of applications of the sterilization bag is wide.

Next, the third embodiment of the present invention will be explained.

The shaping method for the gas-permeable material, the synthetic resin film, and the bag used in this third embodiment is the same as those in the first and second embodiments.

In the present embodiment, the protective component that protects the sterilization paper, which is a gas-permeable material, is arranged in the lower end portion vicinity region of the sterilization bag of the present invention. A material similar to the above-described synthetic resin film material or gas-permeable material can be selected and used for the protective component, and the protective component itself can be either gas-permeable or not.

The protective component can be a molded article made from synthetic resin or metal, but from the standpoint of making the sterilization bag thin, it is preferable that the component be formed as a film. Metallic netting or mesh whose material and/or size is appropriately selected can be used as the material for the protective component that is a film component, as well as sheet material such as synthetic resin film or metal foil.

In considering the gas permeability of the protective component, for example, for a sterilization bag using EOG and the like, forced gas interchange is performed. Therefore, gas permeability is not especially necessary for the protective component. However, in cases where sterilization methods using high-temperature steam or plasma and the like are applied to the sterilization bag, it is preferable to use a gas-permeable film for the protective component. The gas-permeable film can be a regular synthetic resin film provided with minute openings or a metallic netting or mesh, as well as paper or non-woven fabric.

The molded article can be one, which is made of a material such as polyethylene or polypropylene, which has an opening in the upper portion thereof, and which has a closed bottom or a bag shape. That is, when viewed from the cross-section thereof, this is formed into a U shape or an L shape.

The sheet material is not particularly limited, as long as it has strength enough to endure piercing from the contents. Examples of such a material include synthetic resin film, metal foil, metallic netting, and nets braided or woven from synthetic resin filaments and synthetic fibers.

For example, the gas-impermeable sheet-shaped protective component can be polyethylene or polypropylene film having a thickness of about 50 to about 200µm. Moreover, the gas-permeable, sheet-shaped protective component is preferably non-woven fabric made from polyethylene or polypropylene and having a weight of 5 to 100g/m² and preferably 20 to 60 g/m², because it excels in heat-sealing strength. Further, both can be combined to form the protective component.

The protective component in the present embodiment protects at least the inner surface of the gas-permeable material. However, it can protect not only the gas-permeable material, but also the synthetic resin film, and the adhered portion of the gas-permeable material and the synthetic resin film.

When it protects only the gas-permeable material, the protective component can be one sheet of film. When it also protects the surface of the synthetic resin film, two sheets of protective component formed into film can be used, or a protective component formed in a tube shape, or a protective component formed into a bag shape by mountain folding one sheet of film can also be used.

The protective component is fixed to the interior side of the sterilization bag. It is preferable that the protective component is fixed to at least the gas-permeable material by heat sealing or adhering and the like.

The protective component can be formed with a component separate from the two side surface sheets (gas-permeable material and synthetic resin film), such as a third sheet of synthetic resin film. However, the synthetic resin film can be extended and the extended portion can also be used as the protective component.

When the protective component is fixed to the interior of the sterilization bag, it is preferable that the upper end of the protective component (i.e., the end of the protective component which end is on the opening end portion side of the sterilization bag) be closely adhered by heat sealing or adhesion to the side surface film of the sterilization bag. By this embodiment, the catching of objects to be sterilized on the end portion of the protective component can be suppressed.

A protective component extended from the synthetic resin film, or a protective component formed in a bag shape by mountain folding the third synthetic resin film sheet into an L shape or U shape is arranged on and adhered to the interior side of the sterilization bag, whereby the bag-shaped protective component is formed and fixed. At this time, by sandwiching both side ends of the protective component between both side ends of the sterilization bag and adhering these side ends of the protective component to those of the sterilization bag, the protective component becomes fixed more certainly and also protects the heat-sealed portions in both side ends of the sterilization bag.

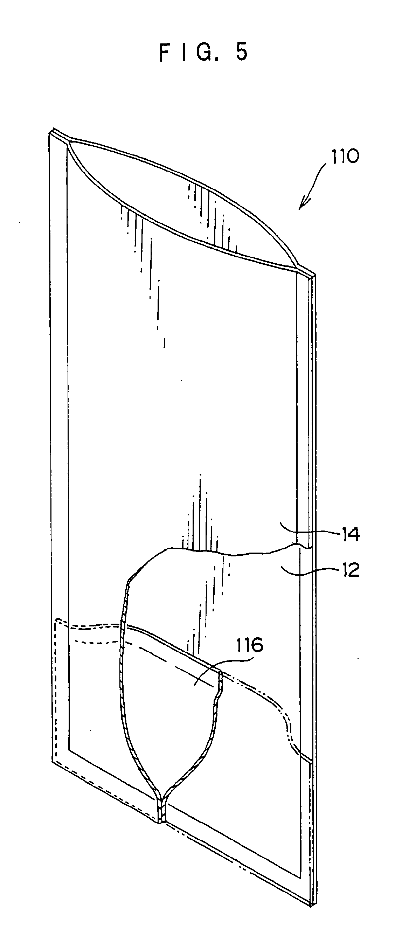

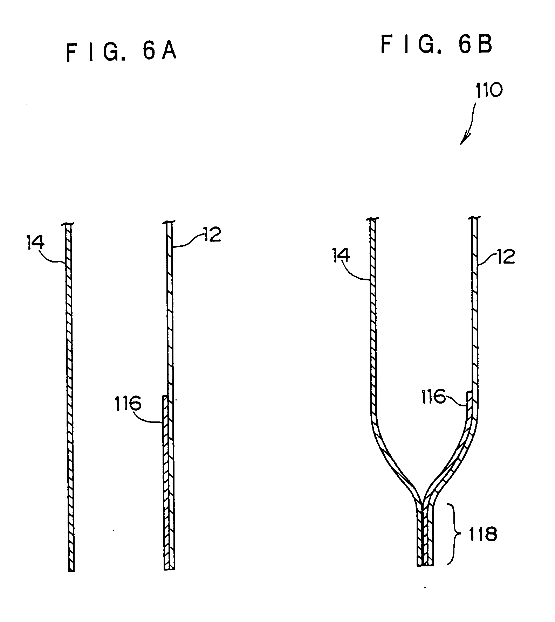

Fig. 5 is a perspective diagram showing one preferable example of the lower end portion vicinity of the sterilization bag of the third embodiment of the present invention. In order to clarify the laminated state of each of the films of the sterilization bag, a partial cutaway section has been included in Fig. 5. Further, Fig. 6A is a diagram for clarifying the configuration thereof and shows a cross-sectional diagram of the films forming the sterilization bag. Fig. 6B shows a cross-sectional diagram of the sterilization bag formed by heat sealing these films.

The gas-permeable material 12 of a sterilization bag 110 is made from sterilization paper, and the other of side surface materials of the sterilization bag 110 is a synthetic resin film 14 having heat-sealing property with at least the innermost layer of polyethylene and polypropylene and the like. In the lower end portion vicinity of the sterilization paper 12, a film-shaped protective component 116 of synthetic resin having heat-sealing property with both surfaces of the sterilization paper 12 which are made from polyethylene or polypropylene and the like is laminated on the sterilization paper 12. The upper end of the sterilization bag 110 (not shown) forms an opening to enable the insertion of objects to be sterilized. As shown in Fig. 6B, the lowermost end portion of the sterilization bag 110 is sealed by heat sealing the sterilization paper 12 and the synthetic resin film 14 through the protective component 116. An unsealed portion may also be left in the lowermost end of the sterilization bag 110.

This protective component 116 is fixed to the sterilization paper 12 by heat sealing. It is sufficient that the upper end of the protective component 116, that is, the distal end portion of the protective component which distal end portion is on the upper opening portion side of the sterilization bag, is closely fixed to the sterilization paper 12 so as to have no air spaces between them. That is, it is not absolutely necessary for the entire surface of the protective component 116 to be adhered to the sterilization paper 12. It is preferable that both end portions of the protective component 116 are fixed by heat sealing to the sterilization paper 12 and the synthetic resin film 14, respectively. However, it is unnecessary to fix both.

In the sterilization bag 110 of the present embodiment, the lower end portion of the sterilization bag 110 and vicinity thereof, that is, the region where inserted objects to be sterilized come into direct contact with the sterilization bag 110 are formed with synthetic resin film 14 and the protective component 116 which have high surface strength and smoothness. Therefore, even when objects to be sterilized with tips having sharp forms are inserted into the sterilization bag 110, there is no risk of the tips or uneven portions of the inputted objects to be sterilized sticking in the surface of the sterilization paper 12.

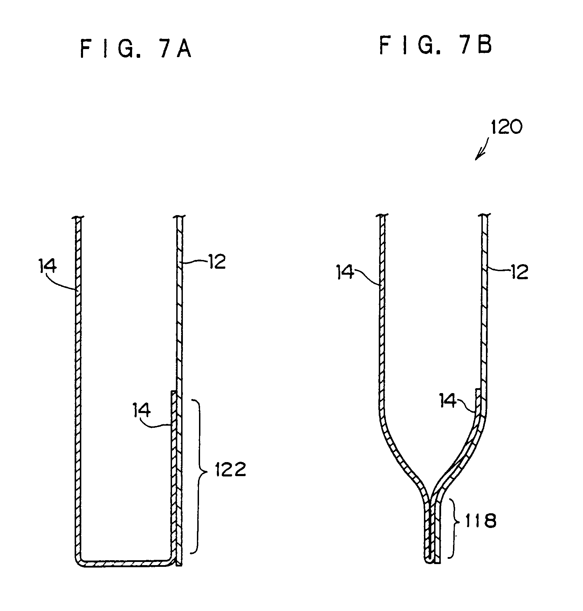

Fig. 7A is a cross-sectional diagram showing another preferable example of the lower end portion vicinity of a sterilization bag 120 of the present invention, and it shows a cross-sectional view of the films forming the sterilization bag. Fig. 7B shows a cross-sectional view of the sterilization bag formed by heat sealing these films.

In the sterilization bag of this example, the side surface film 14, both surfaces of which are made from synthetic resin having heat sealing property, such as polyethylene or polypropylene, is extended and mountain folded to form a bag-shaped protective component.

The sterilization paper that is the gas-permeable material 12, and the synthetic resin film 14 are adhered to each other to form an adhered portion whose upper end line is parallel to the lower end line thereof and which has a predetermined width. The adhered portion is shown in Fig. 7A as 122. After adhesion, the synthetic resin film 14 is mountain folded so that the non-adhered portion thereof also overlaps with the gas-permeable material 12. As shown in Fig. 7B, the lowermost portion is sealed by heat sealing the gas-permeable material 12 and the synthetic resin film 14 through the synthetic resin film adhered to the gas-permeable material 12 (functioning as the protective component 116 in Fig. 6B). The lowermost end is a heat-sealed portion 118. The lowermost end can have a non-sealed portion remaining. The extended end portion of the synthetic resin film 14 functioning as the protective component is fixed to the gas-permeable material by heat sealing.

In this example, it is not necessary to provide a separate part serving as the protective component 116. This is due to the fact that the protective film region can be formed by extending the synthetic resin film 14. Thus, this example has the advantageous point that the sterilization bag can be manufactured through a simple process.

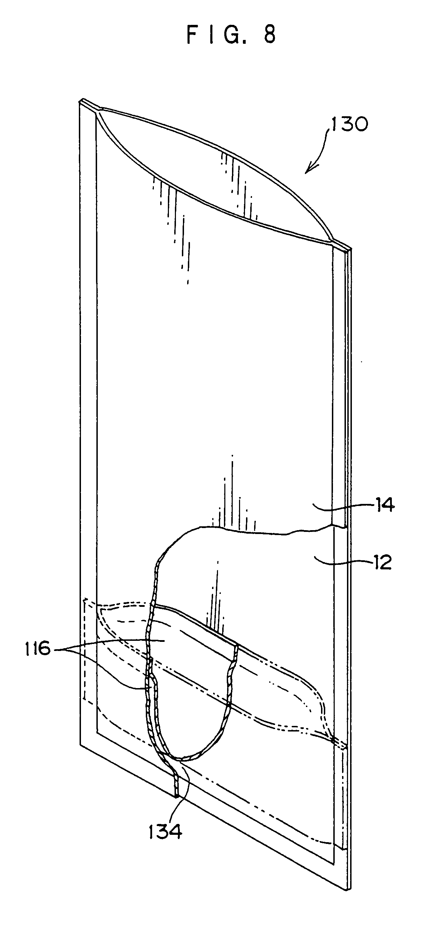

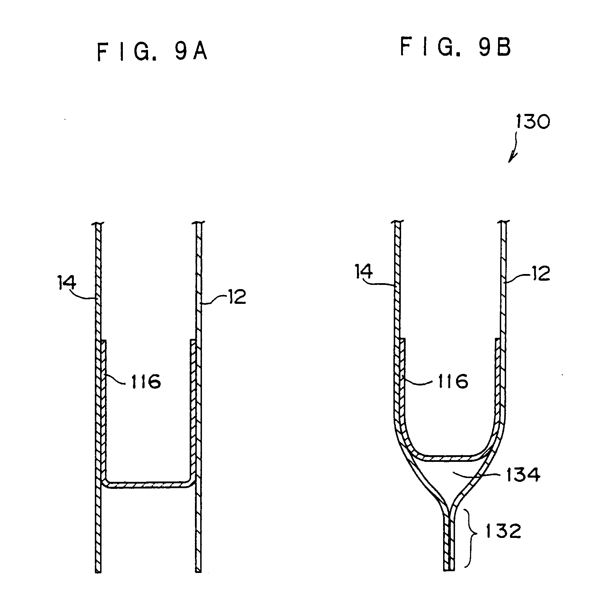

Fig. 8 is a perspective diagram showing another preferable example of the lower end portion vicinity of the sterilization bag of the third embodiment of the present invention, and in order to clarify the laminated state of each of the films of the sterilization bag, a partial cutaway portion has been included in Fig. 8. Fig. 9A is a drawing that clarifies this configuration and shows a cutaway view of the films forming the sterilization bag, and Fig. 9B shows a cross-sectional view of the sterilization bag 30 formed by heat sealing these films.

The side surface films (i.e., the sterilization paper 12 and synthetic resin film 14) used in the sterilization bag 130 can be the same as those of the above two examples of the third embodiment. In the present example, the protective component 116 is a bag-shaped portion formed facing the exterior side. At least both end portions of the bag-shaped protective component 116 are fixed by heat sealing to the sterilization paper 12 and the synthetic resin film 14, respectively. In the present example, the synthetic resin side surface film 14 and the sterilization paper 12 only are sealed at the sealed portion 132 which is the lower end of the sterilization bag 130, and an air space 134 is formed between the lower end of the bag-shaped portion of the protective component 116 and the sealed portion 132. In the present example as well, similar to the above two examples shown in the third embodiment, the protective component 116 is positioned on the interior surface of the bag 130 to protect especially the surface of the sterilization paper 12. Even if objects to be sterilized having sharp tips are inputted in the sterilization paper 130, there is no risk of the tips or uneven portions lacerating the sterilization paper 12 due to the surface strength and smoothness of the protective component 116 made from synthetic resin film. Further, the air space 134 existing between the lower end sealed portion 132 of the sterilization bag 130 and the bottom portion of the bag-shaped protective component 116 serves to function as a cushion that eases the impact received at the sealed portion 132 by the objects to be sterilized being inputted in the sterilization bag 130. For this reason, the durability of the sterilization bag 130 improves. In the present example, the protective component 116 can be a single layer synthetic resin film-type protective component of polyethylene, polypropylene or a non-woven fabric and the like where both surfaces have heat-sealing property. However, use of a protective component where an oriented film is laminated with the above film and where only one surface has heat-sealing property is preferable from a manufacturing standpoint, because this can be manufactured by converting a conventional standing pouch bag-producing machine.

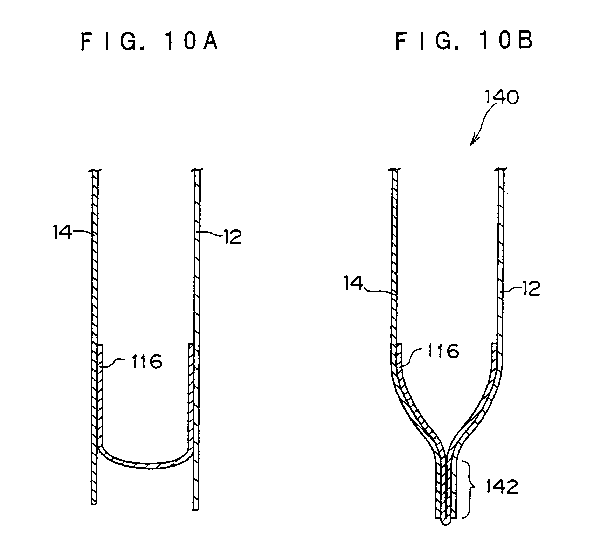

Fig. 10A shows a cross-sectional view of the films forming the sterilization bag which is a fourth variant example of the third embodiment, and Fig. 10B is a cross-sectional view of a sterilization bag 140 formed by heat adhering these films.

In the fourth example of the third embodiment, the sterilization paper 12 and the synthetic resin film 14 used in the sterilization bag 140 can be the same as those in each of the other examples of the third embodiment. In the present example, it is preferable that the protective component 116 be a single layer synthetic resin film-type protective component made of polyethylene, polypropylene, or a non-woven fabric and the like whose both surfaces have heat-sealing property. In the present example, the protective component 116 is similar to the third example in that it faces the exterior side and is formed into a bag, however, the length of an end portion of each of the side surface films 12 and 14 relative to the bag-shaped protective component 116 is approximately equal to the length of the mountain folded portion of the protective component 116. These are approximately equal in the present example and this example is preferable, however, these can also be different. At least both end portions of the protective component 116 are respectively fixed by heat sealing to the sterilization paper 12 and the synthetic resin film 14. In this fourth example, the synthetic resin film 14 and the sterilization paper 12 are sealed through the mountain folded portion of the protective component 116 that exists between them, thus forming a heat sealed portion 142 at the lower end of the sterilization bag 140. In the present example as well, the protective component 116 is positioned on the interior surface of the bag 140 to protect especially the surface of the sterilization paper 12. Even if objects to be sterilized having sharp tips or uneven portions are inputted into the sterilization bag 140, there is no risk of the points lacerating the sterilization paper 12 due to the surface strength and smoothness of the protective component 116 made from synthetic resin film. Further, in the lower end sealed portion 142 of the sterilization bag 140, a strong heat-sealed portion 142 is formed by heat sealing the four layers: the bottom portion of the bag-shaped protective component 116 (two-layer configuration) and the two side surface films, i.e., the sterilization paper 12 and synthetic resin film 14. For this reason, even if the objects to be sterilized inputted in the sterilization bag 140 directly come up against the sealed portion 142, there is no risk of peeling and the like occurring, and the durability of the sterilization bag 140 increases. A non-sealed portion can be left in the region that does not hinder the sterilization effect at the lowermost end of the sterilization bag 140.

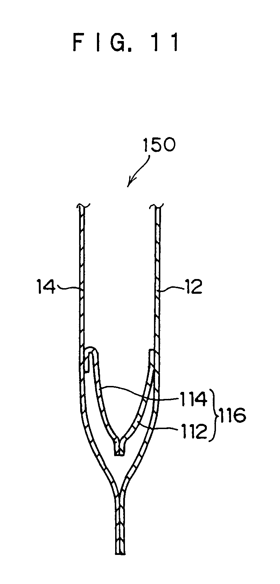

Fig. 11 is a fifth variant example of the third embodiment showing a cross-sectional view of a lower end portion vicinity of a sterilization bag 150.

The sterilization paper 12 and the synthetic resin film 14 used in the sterilization bag 150 of fifth variant example of the third embodiment can be the same as those used in each of the other examples of the third embodiment. In the present example, the protective component 116 is a V-shaped bag component formed by heat sealing one end of a synthetic resin film 114, which is a laminated body of a high-strength synthetic resin film such as polyester and a synthetic resin film having heat-sealing property such as polypropylene; and one end of sterilization paper 112, which is a non-woven fabric made of polypropylene having heat-sealing property. By selecting materials having heat-sealing property as the synthetic resin materials for the synthetic resin film 114 and the non-woven 112, a strong heat-sealed portion is obtained. Therefore, if the tips of tweezers and the like are received in the protective component 116, peeling does not easily occur. Further, even if a situation were to occur such as a portion peeling, the effect on the lower end sealed portion of the sterilization bag 150 is extremely small. This is because an air space exists between the lower end sealed portion of the sterilization bag 150 and one end portion of the V-shaped bag protective component 116 (heat-sealed portion).

In the present example, by using the protective component 116 obtained by heat sealing the non-woven fabric 112 and the synthetic resin film 114, the protective component exhibits superior in gas permeability to a protective component made only from synthetic resin film. Because it does not hinder the penetration of steam or gas used in sterilization, reduction of the sterilization effect can be suppressed. Moreover, the synthetic resin film laminated body 114 is transparent, and provides superior visibility. Thus, it is advantageous in that the contents can be easily confirmed and it is highly safe.

Regarding visibility, there are various shapes of the tips of tweezers, which are objects to be sterilized. However, the grip portions in these are usually all similar. Therefore, it is difficult to confirm the shapes of the tips if a non-transparent material is used for the protective component 116. The high visibility of the tips due to the present example is useful from the standpoint of being able to select instruments without error during medical treatment, and it is easy to confirm whether the tips of the instruments have a defect. Moreover, being able to clearly see the tips of the objects to be sterilized is important for the safety of the user.

In the third embodiment of the sterilization bag of the present invention, the surface of the sterilization paper, which has air spaces between the fibers (to which there is a risk of tip portions of objects to be sterilized entering when objects to be sterilized are inserted into the sterilization bag) is protected by a protective component, typically a protective film. For this reason, when objects to be sterilized having sharp tips such as large tweezers, forceps, and scissors are inserted and retained in the sterilization bag, damage such as unwanted tearing or generation of pinholes in the bag can be effectively prevented. For this reason, there is a wide range of applicable uses for the sterilization bag.

### Examples

Hereafter, the present invention will be explained in detail with reference to specific examples, however, the present invention is not limited thereto.

### Example 1

A synthetic resin film 14 and a gas-permeable substrate (side surface film) 12 were prepared. The gas-permeable substrate (side surface film) 12 was a high-density polyethylene non-woven cloth having a width of 300mm. The synthetic resin film 14 was composed of a biaxially oriented PET film having a thickness of 12µm, a biaxially oriented nylon film having a thickness of 15µm, and a PP film having a thickness of 30µm. One end portion of the synthetic resin film 14 was folded to form a bulging portion 16 which had an N-shape cross section and whose height (width of the overlapping portion of the folded portion) was 40mm. Thereafter, the synthetic resin film 14 was disposed on the gas-permeable substrate 12 so that an end of the synthetic resin film 14 serving as an end (unfolded end) of the bulging portion coincided with one end of the gas permeable substrate 12 and so that the distance between the other end (folded end) of the bulging portion and the one end of the gas permeable substrate 12 became 50mm. Then, the one end of the gas permeable substrate 12 and the unfolded end of the bulging portion were heat sealed with a 10mm heat-seal bar. Next, both side ends of the bulging portion were obliquely heat sealed at a width of 10mm so that the intersecting angle between each of the side ends of the bulging portion and the folding line of the mountain folded synthetic resin film became 45°. After that, the side edge portions of the synthetic resin film 14 and the gas-permeable substrate 12 were heat sealed so that 10mm heat-sealed portions perpendicular to the folding line were formed and so that the inner width between the heat-sealed portions became 160mm. A center of the heat-sealed portions of the side edges were cut into individual bags and the exterior sides of the oblique sealed portions of the bulging portion were cut and removed, and a sterilization bag 10, as shown in Fig. 1, having a width of 170mm and a height of 300mm was obtained.

The opening end of this sterilization bag 10 was opened by hand, and two forceps having sharp tips were put into the sterilization bag 10. As a result, the forceps were stored in the sterilization bag 10 so that the tips contacted the folding line of the mountain folded bulging portion which was a part of the synthetic resin side surface film. Damage to the sterilization bag due to this insertion was not observed.

### Example 2

A gas-permeable substrate 12 made of sterilization paper and having a width of 70mm, a height of 350mm and a weight of 70g/m², and a synthetic resin film 14 having the same measurements as those of the gas-permeable substrate 12 and composed of a biaxially oriented PET film having a thickness of 12µm and a PP film having a thickness of 30µm were used. A polypropylene non-woven fabric having a width of 70mm, a height of 45mm and a weight of 40g/m² was used as the protective component 116. The protective component was disposed on one end portion of the gas-permeable substrate 12 so that the protective component 116 and the one end portion of the gas-permeable substrate 12 coincided with each other. The entire surface of the overlapping portion was heat sealed.

The synthetic resin film 14 and the gas-permeable substrate 12 were layered, with the protective component 116 sandwiched between them. A heat-sealed portion having a width of 10mm was formed in the lower end portion of the layered article. Moreover, heat-sealed portions each having a width of 5mm were formed in the side portions thereof. Thus, a sterilization bag 110, as shown in Fig. 5, with a width of 170mm and a height of 350mm was obtained.

The opening end of this sterilization bag 110 was opened by hand, and the bag was inclined, and large tweezers having sharp tips and a weight of 80g and a length of 25cm was inserted from the opening end into the bag. The tweezers slid on the interior side surface of the gas-permeable substrate and the tips stopped at the region having the protective component 116. No damage to the sterilization bag due to this insertion was observed. This insertion was repeated with ten samples, however, no damage to the sterilization bag, such as the occurrence of pinholes caused by the tips catching or piercing, was observed for any of the samples.

### Example 3

A sterilization bag 130 having a width of 170mm and a height of 300mm, as shown in Fig. 8, was produced in the same manner as in Example 2, except that the following points were changed. That is, the width and height of the protective component 116 were 70mm and 60mm, respectively. The protective component 116 was folded in two so that the height became half. One end portion of the protective component 116 was disposed on a gas-permeable substrate 12, and the other end portion thereof was disposed on a synthetic resin film 14 so that the distance between the folding line of the protective component 116 and each of the lower end portions of the gas-permeable substrate 12 and the synthetic resin film 14 was 15mm. Then, only both ends of the protective component 116 were respectively heat sealed to the gas-permeable material 12 and the synthetic resin film 14 to form heat-sealed portions having a width of 5mm. A heat sealed portion disposed at the lower end portion of the bag was composed of only the gas-permeable material 12 and the synthetic resin film 14. Moreover, the width of the heat sealed portion was 5mm.

The opening end of this sterilization bag 130 was opened by hand and the sterilization bag was inclined, and large tweezers having sharp tips and a weight of 80g and a length of 25cm was inserted from the opening portion into the bag. These slid on the interior side surface of the gas-permeable substrate and the tips stopped at the region having the protective component 116. No damage to the sterilization bag due to this insertion was observed. This insertion was repeated with ten samples, however, no damage to the sterilization bag, such as the occurrence of pinholes caused by the points catching or piercing, was observed for any of the samples.

### Possible Industrial Uses

The sterilization bag of the present invention exhibits superior handling, and can suppress damage and unwanted opening accidents when storing objects having sharp point portions to be sterilized such as forceps, large tweezers, scalpels and scissors. Therefore, it can be favorably used for storing and sterilizing such sharp-pointed medical instruments.

## Claims

1. A sterilization bag **characterized in that** side edges and lower ends of a gas-permeable material and a synthetic resin film are adhered with an opening remaining in an upper end thereof and
which has a damage prevention means that prevents damage of at least one selected from the gas-permeable material, the synthetic resin film and an adhered portion of the gas-permeable material and the synthetic resin film by catching or receiving the tips of stored articles in the lower end portion of the sterilization bag or vicinity thereof.

2. The sterilization bag of claim 1, wherein the damage prevention means is a bulging portion obtained by mountain folding and valley folding the synthetic resin film and prevents the damage by receiving the tips of stored articles.

3. The sterilization bag of claim 1, wherein the damage prevention means is a bulging portion formed by mountain folding the synthetic resin film in a lower portion of the sterilization bag, and the folding line of a side surface film mountain folded is positioned at the lowermost end of the sterilization bag.

4. The sterilization bag of claims 2 or 3, wherein the side edges of the bulging portion is adhered obliquely so that the lower end becomes shorter than the opening in the upper end.

5. The sterilization bag of claims 2 or 3, wherein the intersecting angle of an adhered portion in the side edge of the bulging portion and the folding line of the side surface film made of synthetic resin mountain folded is in the range of 120° to 150°.

6. The sterilization bag of claims 4 or 5, wherein the out side of the side edge of the bulging portion is cut and removed.

7. The sterilization bag of claim 1, wherein the damage prevention means is a protective component disposed in the inner surface of the lower end portion of the sterilization bag or the vicinity thereof, and prevents the damage by catching the tip of stored articles.

8. The sterilization bag of claim 7, wherein the protective component is a filmy protective component.

9. The sterilization bag of claim 8, wherein the protective component is formed by extending and folding the synthetic resin film.

10. The sterilization bag of claims 7 or 8, wherein the protective component is a bag component formed by mountain folding a third synthetic resin film.

11. The sterilization bag of any one of claims 7, 8 and 10, wherein the protective component is made of a material having gas permeability.

12. The sterilization bag of claim of any of claims 1 to 11, wherein the sterilization bag is used for sterilizing one or two or more kinds of medical instruments selected from the group consisting of scalpels, tweezers, forceps and scissors.
